(19) Europäisches Patentamt
European Patent Office
Office européen des brevets

(11) EP 4 342 453 A1

(12) EUROPEAN PATENT APPLICATION
published in accordance with Art. 153(4) EPC

(43) Date of publication:
**27.03.2024 Bulletin 2024/13**

(21) Application number: **22853492.1**

(22) Date of filing: **04.08.2022**

(51) International Patent Classification (IPC):
***A61K 9/00*** (2006.01) ***A61K 9/08*** (2006.01)
***A61K 47/26*** (2006.01) ***A61K 47/10*** (2017.01)
***A61K 47/44*** (2017.01) ***A61K 31/352*** (2006.01)
***A61P 27/02*** (2006.01) ***A61P 27/04*** (2006.01)

(52) Cooperative Patent Classification (CPC):
**A61K 9/00; A61K 9/08; A61K 31/352; A61K 47/10; A61K 47/26; A61K 47/44; A61P 27/02; A61P 27/04**

(86) International application number:
**PCT/KR2022/011567**

(87) International publication number:
**WO 2023/014117 (09.02.2023 Gazette 2023/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.08.2021 KR 20210103200**

(71) Applicant: **GL Pharmtech Corp.**
**Gyeonggi-do 13449 (KR)**

(72) Inventors:
- **KIM, Eun Jung**
  **Seongnam-si Gyeonggi-do 13449 (KR)**
- **PARK, Jun Sang**
  **Seongnam-si Gyeonggi-do 13449 (KR)**

(74) Representative: **Louis Pöhlau Lohrentz**
**Patentanwälte**
**Postfach 30 55**
**90014 Nürnberg (DE)**

(54) **EYE DROP COMPOSITION FOR TREATING DRY EYE SYNDROME CONTAINING RECOFLAVONE AND METHOD FOR PREPARING SAME**

(57) The present invention relates to an eye drop composition for treating dry eye syndrome, containing recoflavone.

Particularly, the present invention relates to an eye drop composition which, by containing a certain concentration or higher of a main ingredient in the composition and comprising one or more permeation enhancers, so that the active ingredient recoflavone is sufficiently delivered to corneal epithelial cells in vivo to exhibit an effective medicinal effect, can maximize the amelioration of dry eye symptoms.

【FIG. 1】

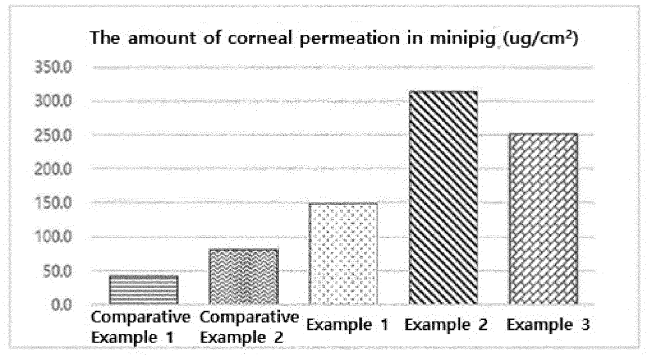




Processed by Luminess, 75001 PARIS (FR)

## Description

### [Technical Field]

The present invention relates to an ophthalmic composition containing recoflavone for the treatment of dry eye disease, which is a 3',4',5-trimethoxyflavone derivative compound and a method for preparing the same. Specifically, in order to maximize the improvement of symptoms of dry eye, the present invention relates to an ophthalmic composition containing a certain concentration of the active ingredient and at least one of permeation enhancers, and a method for preparing the same, so that the active ingredient, recoflavone, can sufficiently penetrate the cornea *in vivo* to exhibit significant therapeutic effects.

### [Background Art]

Dry eye disease is known as a disease that causes inflammation on an ocular surface and discomfort in eyes due to an increased osmolarity of the tear film and loss of tear homeostasis caused by tear deficiency or excessive tear evaporation. However, there was much controversy regarding the appropriate definition for dry eye until only a few years ago, the Tear Film and Ocular Surface Society (TFOS) defined dry eye disease as follows in 2017: "Dry eye disease is a multifactorial disease of the ocular surface characterized by a loss of homeostasis of the tear film, and accompanied by ocular symptoms, in which tear film instability, hyperosmolarity, inflammation and damage of ocular surface, and neurosensory abnormalities play etiological roles. (TFOS DEWS II introduction. Ocul. Surf. 2017, 15, 269-275)." The diagnosis of dry eye disease is performed by using such methods as questionnaires about symptoms, tear volume, tear film breakup time, tear osmolarity, keratoconjunctival staining and the analysis of lipid layer or meibomian gland, and depending on the degree of dry eye disease, artificial tears, anti-inflammatory agent, tear secretion promoters, autologous serum and eyelid treatment are used for the treatment (J. Korean Med. Assoc. 2018, 61(6), 352-364).

For the treatment of dry eye disease, tear replacement approaches with ocular lubricants such as artificial tears are most commonly used and typical examples thereof are eye drops containing sodium hyaluronate or sodium carboxymethylcellulose. For anti-inflammatory therapy, a cyclosporine formulation is used to reduce the immunoactivity markers (e.g., human leukocyte antigen-antigen D related), apoptosis markers (e.g., Fas) and inflammatory cytokines (e.g., interleukin-6) in keratoconjunctival epithelium to treat inflammation on the ocular surface and improve the symptoms of dry eye. The recently developed diquafosol (diquafosol tetrasodium) formulation is a P2Y2 receptor agonist and exhibits the promotion of secretion of aqueous and mucous components of tear. In Japan, a rebamipide suspension formulation has been proven to be effective in increasing the density of goblet cells in eyes and increasing the mucous and aqueous components of tear, and is used as a therapeutic agent for dry eye disease.

Recoflavone is a 3',4',5-trimethoxyflavone derivative compound, $C_{20}H_{18}O_8$, and has a molecular weight of 386.4 by 2-((2-(3,4-dimethoxyphenyl)-5-methoxy-4-oxo-4H-chromen-7-yl)oxy) acetic acid.

Korea Patent Registrations Nos. 10-0447918, 10-0327621 and 10-0644928 disclosed the applicability of recoflavone to gastrointestinal diseases such as gastritis and gastric ulcer, and inflammatory bowel diseases such as Crohn's disease and colitis by the anti-inflammatory effect and the promotion of gastric mucus secretion. Korea Patent Registration No. 10-0930467 disclosed the conjunctival mucus secretion promoting action of 3',4',5-trimethoxyflavone derivative compounds including recoflavone and the applicability as an agent for treating and preventing dry eye disease.

Korea Patent Registration No. 10-0644928 described that recoflavone can be used in the form of an anhydride as it is, but since its hygroscopicity causes problems in handling and management, it can be improved when it is used as a hydrate.

Recoflavone is practically insoluble in water. It has a property with low solubility at low pH. As the pH increases, it is ionized and its solubility increases. However, even when it was dissolved, recoflavone exhibits poor cell permeability due to the high degree of ionization and it could have a problem in the absorption into the body. In fact, when a 3% eye drop was administered to rabbit eyes and the drug distribution in each tissue of eye with time was evaluated, the results showed that recoflavone was rarely distributed in the tissues located inside the eye and mainly distributed in the surrounding tissues outside of the eye, such as the cornea, conjunctiva, and sclera. In particular, the results showed that the drug concentration in the cornea was high immediately after the administration, but it was rapidly decreased over time and the drug was mainly distributed in the conjunctiva and sclera (data not shown).

Therefore, it needs that an eye drop formulation of recoflavone can penetrate the keratoconjunctiva *in vivo* in a certain amount or more and a method for preparing the same so that it can exhibit significant therapeutic effects on the overall symptoms of dry eye disease.

[Prior Arts]

[Patent Document]

(Patent Document 1) Korea Patent Registration No. 10-0327621
(Patent Document 2) Korea Patent Registration No. 10-0447918
(Patent Document 3) Korea Patent Registration No. 10-0644928
(Patent Document 4) Korea Patent Registration No. 10-0930467

[Non-patent Document]

(Non-patent Document 1) TFOS DEWS II introduction. Ocul. Surf. 2017, 15, 269-275
(Non-patent Document 2) J. Korean Med. Assoc. 2018, 61(6), 352-364
(Non-patent Document 3) Invest. Ophthalmol. Vis. Sci., 2014, 55(10), 6569-6574
(Non-patent Document 4) Cornea, 2017, 36(6), 712-718
(Non-patent Document 5) Br. J. Ophthalmol. 2010, 94, 1519-1522

**[Disclosure of Invention]**

**[Technical Problem]**

The present invention relates to an ophthalmic composition containing recoflavone for the treatment of dry eye disease, and provides an ophthalmic composition of which active ingredients can penetrate the keratoconjunctiva *in vivo* in a certain amount or more and a method for preparing the same so that recoflavone can exhibit significant therapeutic effects on the overall symptoms of dry eye disease.

Korea Patent Registration No. 1 0-0930467 disclosed that recoflavone showed some evidences for the treatment of dry eye disease, such as the promotion of mucus secretion, at a low drug concentration and had the possibility of development as a therapeutic agent of dry eye disease.

Normally, when a scar occurs in the keratoconjunctiva of an eye, the expression of major mucin proteins, such as MUC1, MUC4, MUC16, and MUC5AC, is increased. However, the patients with dry eye disease complain some discomfort because the mucus secretion is not good enough even though it is easy to get scars in their cornea. The pharmacological mucin secretion by recoflavone in human keratoconjunctival epithelial cells was studied and it confirmed that the thickness of the mucin layer was significantly increased when 100 μM solution of the active ingredient was applied. However, real-time Polymerase Chain Reaction (PCR) confirmed that the mucin proteins related with the symptoms of dry eye, which were MUC1, MUC4, MUC16 and MUC5AC, showed a tendency to increase significantly for four hours and then decreased rapidly. The Western blot analysis also showed that MUC5AC only had a tendency to increase and the others did not (Investigative Ophthalmology and Visual Science, 2014, 55(10), 6565-6574). These results suggested that the pharmacological effects of recoflavone could improve only a part of the symptoms of dry eye and the development potential to an efficient therapeutic agent could be restricted when it was considered that dry eye disease has various symptoms caused by various factors.

In the process of developing a formulation containing recoflavone as a therapeutic agent of dry eye disease, it was confirmed that it was difficult to effectively improve the overall symptoms of dry eye in *in vivo* studies with a low drug concentration in a simply dissolved state. It was found through the present invention that the overall symptoms of dry eye could be effectively improved just when recoflavone could permeate into tissues and cells *in vivo* in a certain amount or more. Then it could be developed as a practical therapeutic agent of dry eye disease. Therefore, in the present invention, it is necessary to develop a recoflavone-containing formulation and a method for preparing the same that can efficiently penetrate the cornea to improve the overall symptoms of dry eye significantly in tissues and cells of the eye.

**[Solution to Problem]**

The present invention provides an ophthalmic composition containing recoflavone which efficiently penetrates into the keratoconjunctiva to exhibit significant improvement of the symptoms of dry eye in tissues and cells of the eye. Specifically, the present invention provides an ophthalmic composition wherein:

(a) the effective concentration of recoflavone or a pharmaceutically acceptable salt thereof, a hydrate, anhydride or solvate thereof in the eye drop is 5% (w/v) or higher; and
(b) the ophthalmic composition comprises a permeation enhancer in an amount of 0.1 to 5.0 % (w/v); and a tonicity agent in an amount of 0.01 to 1.0% (w/v).

In addition, the present invention provides an ophthalmic composition for the treatment of dry eye disease, in which the active ingredient, recoflavone, can be dissolved transparently. Specifically, the present invention provides an ophthalmic composition, comprising:

(a) recoflavone, a hydrate, anhydride or solvate thereof in an amount of 5% (w/v) or more; and
(b) alkalizing agents, wherein the ratio of molar concentration of the alkalizing agent to that of recoflavone is 1.05 or higher.

In addition, the present invention provides a method for preparing an ophthalmic composition containing recoflavone which efficiently penetrates into the keratoconjunctiva to exhibit significant improvement of the symptoms of dry eye in tissues and cells of the eye. Specifically, the method comprises:

(S1) adding and dissolving recoflavone, a hydrate, anhydride or solvate thereof into the solution prepared by adding and dissolving an alkalizing agent into purified water;
(S2) adjusting pH to a range from 4 to 8 by adding an acidifying agent to the solution obtained through (S1);
(S3) adding and dissolving at least one pharmaceutical additive consisting of a permeation enhancer, a tonicity agent, a buffering agent and a preservative into the solution obtained through (S2); and
(S4) filtering the solution obtained through (S3).

In addition, the present invention provides an ophthalmic composition maintaining pH stability. Specifically, the present invention provides an ophthalmic composition, comprising:

(a) recoflavone or a pharmaceutically acceptable salt thereof, a hydrate, anhydride or solvate thereof in an amount of 5% (w/v) or more;
(b) pH-adjusting agents in an amount of 0.01 to 3.0 % (w/v); and
(c) maintaining the pH in a range from 4 to 8 under long term storage condition (25°C/60%RH).

In addition, the present invention provides the method for administration (usage) of an ophthalmic composition. Specifically, the present invention provides an ophthalmic composition, comprising:

(a) recoflavone or a pharmaceutically acceptable salt thereof, a hydrate, anhydride or solvate thereof in an amount of 5% (w/v) or more; and
(b) a permeation enhancer in an amount of 0.1 to 5.0 % (w/v);
(c) 1 to 2 drops to be administered at a time and at least three times a day.

**[Advantageous Effects of Invention]**

The present invention can effectively improve the symptoms of dry eye disease by increasing the amount of keratoconjunctival permeation *in vivo* of a recoflavone-containing ophthalmic composition for the treatment of dry eye disease. In particular, the present invention provides a practical and efficient ophthalmic composition for the treatment of dry eye disease by confirming the efficacy of the ophthalmic composition of this invention was superior to that of placebo in a clinical study as well as by improving the overall signs of dry eye disease, such as tear secretion, corneal fluorescence staining score, the detachment of corneal epithelial cells, corneal surface irregularity, conjunctival goblet cell density and mucin cell density.

In addition, a commercially applicable composition and a method for preparing the same are provided for a transparent ophthalmic composition containing 5% (w/v) or more of recoflavone or a pharmaceutically acceptable salt thereof, a hydrate, anhydride or solvate thereof. Surprisingly, the present invention also provides the stable pH of the ophthalmic composition without a buffering agent under long term storage condition (25°C/60% RH), if necessary.

The effects of the present invention are not limited to the abovementioned effects, and various effects may be included within the range that is obvious to those skilled in the art from the description below.

**[Description of the Drawings]**

FIG. 1 shows the cumulative amount of corneal permeation of recoflavone in the cornea of minipig for 6 hours.
FIG. 2 shows a comparison of tear secretion between the ophthalmic compositions of Comparative Example 1 and Example 1 in the experimental dry eye mouse model.
FIG. 3 shows a comparison of the corneal fluorescence staining score between the ophthalmic compositions of Comparative Example 1 and Example 1 in the experimental dry eye mouse model.
FIG. 4 shows a comparison of the corneal surface irregularity between the ophthalmic compositions of Comparative Example 1 and Example 1 in the experimental dry eye mouse model.
FIG. 5 shows a comparison of the number of detached corneal epithelial cells between the ophthalmic compositions of Comparative Example 1 and Example 1 in the experimental dry eye mouse model.
FIG. 6 shows a comparison of goblet cell density between the ophthalmic compositions of Comparative Example 1 and Example 1 in the experimental dry eye mouse model.
FIG. 7 shows a photograph comparing the mucin cell density between the ophthalmic compositions of Comparative Example 1 and Example 1 in the experimental dry eye mouse model.
FIG. 8 shows a graph comparing the mucin cell density between the ophthalmic compositions of Comparative Example 1 and Example 1 in the experimental dry eye mouse model.
FIG. 9 is a photograph showing the dissolution state of recoflavone depending on the molar concentration of sodium hydroxide aqueous solution in the process of preparing a composition containing 5% (w/v) of recoflavone as a result of Table 2.
FIG. 10 is a graph showing the amount of change form baseline of the total corneal staining score (TCSS) by fluorescein in the clinical study.
FIG. 11 is a graph comparing the pH stability of Example 5 under long-term storage condition (25° C/60% RH).

**[Best Mode for Carrying out the Invention]**

Hereinafter, the present specification will be described in more detail.

Each description and embodiment disclosed in the present invention may be applied to other descriptions and embodiments for each. In other words, all combinations of the various elements disclosed herein fall within the scope of the present invention. In addition, it cannot be considered that the scope of the present invention is limited by the specific descriptions described below.

The inventors of the present invention figured out in the process of developing a formulation containing recoflavone into a therapeutic agent of dry eye disease that it is difficult to effectively improve the overall symptoms of dry eye in *in vivo* studies with a low drug concentration in a just dissolved state. During the research to develop recoflavone as an effective therapeutic agent of dry eye disease, it was found that when the formulation contains 5% (w/v) or more of recoflavone and 0.1 to 5.0 % (w/v) of a permeation enhancer, the amount of keratoconjunctival permeation *in vivo* is increased to effectively improve the symptoms of dry eye. In addition, the inventors of the present invention completed a commercially applicable composition and a method for preparing the same for a transparent ophthalmic composition containing 5% (w/v) or more of recoflavone or a pharmaceutically acceptable salt thereof, a hydrate, anhydride or solvate thereof. Surprisingly, the present invention also provides the stable pH of the ophthalmic composition containing 5% (w/v) or more of recoflavone or a pharmaceutically acceptable salt thereof, a hydrate, anhydride or solvate without a buffering agent under long term storage condition (25°C/60% RH), if necessary.

Hereinafter, the present specification will be described in more detail.

As used here in the present specification, containing 5% (w/v) or more of recoflavone or a pharmaceutically acceptable salt thereof, a hydrate, anhydride or solvate thereof means containing 5% (w/v) or more as recoflavone, referring to the concentration contained as recoflavone for both the content described as an example and the content that is similar to the description. In addition, the concentration of recoflavone also refers to the concentration as recoflavone.

## Ophthalmic composition for treating dry eye disease

The ophthalmic composition of the present invention comprises 5% (w/v) or more of recoflavone or a pharmaceutically acceptable salt thereof, a hydrate, anhydride or solvate thereof, and permeation enhancers and tonicity agents. This ophthalmic composition may optionally further comprise viscosity agents, buffering agents, pH-adjusting agents and preservatives.

Specifically, the present invention provides an ophthalmic composition for treating dry eye disease, the composition comprising 5% (w/v) or more of recoflavone or a pharmaceutically acceptable salt thereof, a hydrate, anhydride or solvate thereof; 0.1 to 5.0% (w/v) of permeation enhancers; and 0.01 to 1.0% (w/v) of tonicity agents.

Recoflavone as the active ingredient of the present invention may be used in the form of an anhydride or a solvate including hydrates, and may also be used as one of its pharmaceutically acceptable salt.

In the present invention, recoflavone may be prepared directly by a conventionally known manufacturing method, or may be prepared by commercial purchase.

In the present invention, the term "recoflavone" refers to a 3',4',5-trimethoxyflavone derivative compound having the structure of Chemical formula 1 below.

[Chemical formula 1]

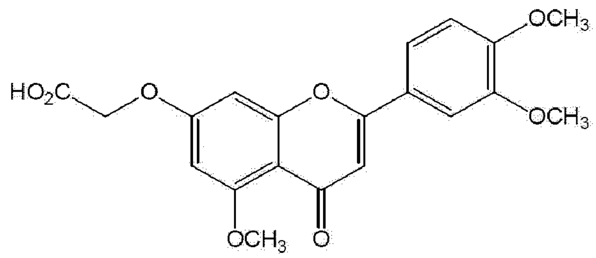

Korean Patent Registration No. 10-0644928 discloses that recoflavone has hygroscopicity in the form of an anhydride as represented by the chemical formula itself, but it is stable without hygroscopicity when it is present in the form of a hydrate, and that it may be present in various solvates. Therefore, it is preferable to use solvates including hydrates in solid dosage forms such as tablet and capsule in the aspect of stability, but in liquid dosage forms such as eye drop, various forms of recoflavone such as anhydrides, solvates including hydrates and its pharmaceutically acceptable salts can be available.

With regard to the concentration of recoflavone, Korean Patent Registration No. 10-0930467 and the prior arts implemented 1 to 3% (w/v) as an active ingredient. However, it was found in the present invention that a certain amount or more of recoflavone must penetrate the tissue *in vivo* in order to improve significantly overall symptoms of dry eye and, in this case, the concentration of recoflavone should be 5% (w/v) or more in a formulation.

Preferably, the concentration of recoflavone is 5% (w/v) or more and 10% (w/v) or less. More than 10% (w/v) of recoflavone is difficult to apply to an eye drop formulation due to osmolarity.

In the present invention, the "permeation enhancer" refers what plays a role of permeating a certain amount or more of recoflavone in the cornea *in vivo* so as to improve significantly overall symptoms of dry eye, when an ophthalmic composition containing recoflavone is administered to the eye, and it may be a kind of surfactant that plays a role of enhancing the cell membrane permeation of recoflavone.

The permeation enhancer may be at least one selected from polysorbate, polyoxyethylene alkyl ether, macrogol 115 hydroxystearate, polyoxyl glyceride, tocopheryl polyethylene glycol succinate, poloxamer and polyoxyethylene castor oil derivatives. Specifically, it may be polysorbate 80, polyoxyl 10 oleyl ether, solutol HS15, lauroyl polyoxyl glyceride, vitamin E-TPGS, poloxamer 407 and polyoxyl 35 castor oil.

In the present invention, the permeation enhancer may be used alone or in combination, and when included alone or in combination, it may be included in a range of 0.1 to 5.0% (w/v) in the ophthalmic composition.

In the present invention, a tonicity agent that is commonly used for isotonicity with tear may be used. For example, nonionic compounds such as mannitol, glycerin, propylene glycol, polyethylene glycol, maltose, sucrose, sorbitol, trehalose and glucose or ionic compounds such as sodium chloride, sodium nitrate and potassium nitrate may be used. These tonicity agents may be used alone or in any combination of two or more thereof to adjust osmotic pressure to be similar to that of tear, and may be included in a range of 0.01 to 1.0% (w/v) in the ophthalmic composition.

In the present invention, the ophthalmic composition for treating dry eye disease may optionally further comprise any one or more of viscosity agents, buffering agents, pH-adjusting agents, and preservatives.

Hereinafter, viscosity agents, buffering agents, pH-adjusting agents, and preservatives are described.

In the present invention, a viscosity agent that is commonly used for eye drops may be optionally used, if necessary. For example, nonionic polymers such as polyvinylpyrrolidone, polyvinyl alcohol, hydroxypropylmethylcellulose, hydroxyethylcellulose, methylcellulose; ionic polymers such as sodium carboxymethylcellulose, polycarbophil, carbomer; sugars such as glucan, chitosan, trehalose; and gums such as xanthan gum may be used, and may be optionally comprised in a range of 0 to 3.0% (w/v) in the ophthalmic composition.

As a buffering agent, phosphate, citrate, acetate, hydrogen carbonate, carbonate, gluconate, lactate, propionate,

TRIS or borate may be used. In this case, buffering agents may be included in a range of 0 to 3.0% (w/v) in the ophthalmic composition, and these may be used alone or in any combination of two or more.

Buffering agents are generally included to obtain stable physicochemical properties, particularly pH, in most ophthalmic compositions for the shelf life of finished products. However, it has been reported in recent studies that, though borate, phosphate, citrate, or TRIS buffers were commonly used for eye drops, they could be toxic when the high concentration of them were exposed to the human corneal-limbal epithelial cells and human conjunctival epithelial cells (Cornea, 2017, 36(6), 712-718). It has been also reported that hyaluronate eye drops comprising a phosphate buffer could induce burning or corneal calcification at corneal wounds (Br. J. Ophthalmol. 2010, 94, 1519-1522).

Therefore, if the pH stability of an eye drop can be secured without a buffering agent, it may be desirable to exclude it. In the case of the ophthalmic composition from the present invention containing 5% (w/v) or more of recoflavone or a pharmaceutically acceptable salt thereof, a hydrate, anhydride or solvate thereof, it was surprisingly found that the pH of the ophthalmic composition without a buffering agent kept stable during the shelf-life. On the other hand, it was also found that the pH of the placebo composition without recoflavone or a pharmaceutically acceptable salt thereof, a hydrate, anhydride or solvate thereof, was decreased continuously.

In the present invention, a pH adjusting-agent that is commonly used for eye drops may be used to adjust and maintain the pH in a range that does not irritate or damage the cornea or conjunctiva. As a pH-adjusting agent, conventional acids such as hydrochloric acid, lactic acid, acetic acid, phosphoric acid, boric acid and citric acid or conventional bases such as sodium hydroxide, potassium hydroxide may be used alone or in any combination of two or more thereof. In this case, the pH adjusting agent and the amount of the same in the ophthalmic formulation may be optionally included so that the pH may be in a range from 4 to 8, or the amount of the pH adjusting agent may be set during the preparation of the composition preferably in a pH range from 5 to 7. The pH adjusting agent may be included in a range of 0.01 to 3.0% (w/v) in the ophthalmic composition.

In the present invention, for the purpose of preventing the eye drop from contamination with bacteria or fungi, a preservative that is commonly used in eye drops may be optionally used, if necessary, and it usually depends on whether a multi-use container or a disposable container is used as a package of an ophthalmic formulation. For example, tertiary ammonium salts such as benzalkonium chloride and benzethonium chloride; paraoxybenzoic acid esters such as methylparaben and propylparaben; alcohols such as chlorobutanol and benzyl alcohol; chlorhexidine acetate and sodium edetate may be used alone or in any combination of two or more thereof, and may be optionally included in a range of 0 to 0.1% (w/v) in the ophthalmic composition.

In the present invention, the ophthalmic composition for the treatment dry eye disease may not comprise a viscosity agent, a buffering agent and/or a preservative, and if not comprised, the lower limit of the content of each ingredient can be expressed as 0% (w/v). In other words, in the present invention, the meaning of 0% (w/v) is that the ingredients may be optionally used, if necessary.

## Improvement of permeation of the ophthalmic composition

In the present invention, for the ophthalmic composition for the treatment of dry eye disease, the cumulative permeation amount of recoflavone in the cornea of minipig for 6 hours is 130 $\mu g/cm^2$ or more, or 3 times or more compared to an ophthalmic composition that does not contain a permeation enhancer. In this case, the cumulative permeation amount of recoflavone means the total amount of recoflavone that has permeated the cornea of minipig for 6 hours when tested according to Experimental Example 1.

Because the cornea has a function of protecting the eyes and acts as a barrier to drug absorption, it is necessary to secure an appropriate corneal permeation amount of drug to successfully develop an eye drop.

Recoflavone has the advantage of being able to prepare an eye drop in a fully transparent solution through the preparation method of the present invention even at a high concentration of 3% (w/v) or 5% (w/v). However, the eye drops containing recoflavone that is just dissolved at a high concentration failed to show a significant improvement of the overall symptoms of dry eye disease in *in vivo* studies, and the results of the corneal permeability test confirmed that recoflavone could not penetrate in a sufficient amount although recoflavone was dissolved at a high concentration. In the present invention, it was confirmed that the corneal permeation amount of recoflavone was significantly increased when a permeation enhancer was included in the recoflavone-containing ophthalmic composition, which induced the superior efficacy of the ophthalmic composition of this invention to that of placebo in a clinical study as well as the improvement of the overall signs of dry eye disease in the experimental dry eye animal model.

In this case, the details mentioned about the ophthalmic composition for the treatment of dry eye disease and the improvement of the permeation of the ophthalmic composition are equally applied as long as they do not contradict each other. The description about the ophthalmic composition for the treatment of dry eye disease can all be applied to the improvement of the permeation of the ophthalmic composition.

## Ophthalmic composition for the treatment of dry eye disease in which the content of an alkalizing agent is adjusted

In addition, the present invention provides an ophthalmic composition for the treatment of dry eye disease in which the active ingredient, recoflavone, can be transparently dissolved.

Specifically, the present invention provides an ophthalmic composition for the treatment of dry eye disease, comprising:

> 5% (w/v) or more of recoflavone or a pharmaceutically acceptable salt thereof, a hydrate, anhydride or solvate thereof; and
> an alkalizing agent, wherein the ratio of molar concentration of the alkalizing agent to that of recoflavone is 1.05 or higher.

In the present invention, the alkalizing agent is sodium hydroxide or potassium hydroxide.

Details about this will be described in the preparation method of the ophthalmic composition for the treatment of dry eye disease.

In this case, the details mentioned about the ophthalmic composition for the treatment of dry eye disease and the ophthalmic composition in which the content of an alkalizing agent is adjusted are equally applied as long as they do not contradict each other. The description about the ophthalmic composition for the treatment of dry eye disease can all be applied to the ophthalmic composition in which the content of an alkalizing agent is adjusted.

## Method for preparing an ophthalmic composition for treating dry eye disease

In addition, the present invention provides a method for preparing an ophthalmic composition for the treatment of dry eye disease that can exhibit effective improvement of the symptoms of dry eye.

Specifically, the method comprises: (S1) adding and dissolving recoflavone, a hydrate, anhydride or solvate thereof into a solution prepared by adding and dissolving an alkalizing agent into purified water; (S2) adjusting pH to a range from 4 to 8 by adding an acidifying agent to the solution obtained through (S1); (S3) adding and dissolving at least one pharmaceutical additive consisting of a permeation enhancer, a tonicity agent, a buffering agent and a preservative into the solution obtained through (S2); and (S4) filtering the solution obtained through (S3).

In the preparation method of the present invention, recoflavone and a hydrate, anhydride or solvate thereof are referred to as recoflavone.

In general, an ophthalmic composition is preferably prepared in a transparent solution state in consideration of the feeling of use in eyes and the efficient absorption of the drug. However, because recoflavone in the form of a hydrate or others is practically insoluble in water as described above, a specific method is required for preparing a transparent solution. Therefore, if recoflavone is not dissolved in purified water for an ophthalmic composition, a transparent solution may be prepared through (S1) step.

In the present invention, the alkalizing agent for pH adjustment in (S1) step is sodium hydroxide or potassium hydroxide, preferably, sodium hydroxide. In (S2) step, the acidifying agent for pH adjustment may be used by selecting one or more from hydrochloric acid, lactic acid, acetic acid, phosphoric acid, boric acid, and citric acid, preferably, one or more selected from hydrochloric acid, phosphoric acid and acetic acid, and most preferably, hydrochloric acid.

When recoflavone is not dissolved in purified water, in (S1) step, sodium hydroxide as an alkalizing agent is added in an amount of not less than 1.05 which is the ratio of molar concentration to that of recoflavone. Here, the ratio of molar concentration is expressed as a value by dividing the molar concentration of sodium hydroxide by the molar concentration of recoflavone contained in the ophthalmic composition.

Specifically, (S1) step is a process where sodium hydroxide is added to purified water to prepare an aqueous solution having a molar concentration of not more than 0.16 M or not less than 0.19 M, and then recoflavone is added. Recoflavone can be dissolved transparently through the above process.

The molar concentration of sodium hydroxide is calculated in the above dissolving process not in the final volume of the manufacturing process. Surprisingly, when recoflavone was added in an aqueous solution of sodium hydroxide having a molar concentration out of the range of not more than 0.16 M or not less than 0.19 M, recoflavone was not dissolved transparently even after stirring for several hours. Therefore an ophthalmic composition cannot be prepared efficiently. The corresponding contents will be described in details with reference to Tables 1, 2 and FIG. 9 below.

In this case, the amount of an alkalizing agent, particularly sodium hydroxide, may be adjusted within the available range in consideration of the osmolarity of an ophthalmic composition and so the ratio of molar concentration by 2.0 or higher is not used.

[Table 1]

| Molar concentration ratio | Molar concentration of sodium hydroxide aqueous solution (M) | Molar concentration of recoflavone monohydrate aqueous solution (M) | Dissolved state |
|---|---|---|---|
| 0.93 | 0.196 | 0.211 | Not transparent |
| 0.97 | 0.196 | 0.201 | Not transparent |
| 1.00 | 0.196 | 0.196 | Not transparent |
| 1.05 | 0.194 | 0.184 | Transparent |
| 1.06 | 0.203 | 0.192 | Transparent |

[Table 2]

| Final volume | Recofla vone concentration (w/v) | Molar concentration of sodium hydroxide aqueous solution (M) | Volume of purified water (mL) | Dissolved state of recoflavone | Molar concentration ratio of sodium hydroxide |
|---|---|---|---|---|---|
| 100 mL | 3 % | 0.086 | 90 | Transparent | 1.05 |
| | | 0.097 | 80 | Transparent | - |
| | | 0.111 | 70 | Transparent | - |
| | 5 % | 0.137 | 100 | Transparent | 1.05 |
| | | 0.152 | 90 | Transparent | - |
| | | 0.172 | 80 | Not transparent | - |
| | | 0.183 | 75 | Not transparent | - |
| | | 0.196 | 70 | Transparent | - |
| | | 0.392 | 70 | Transparent | - |
| | 6 % | 0.181 | 90 | Not dissolved | 1.05 |
| | | 0.204 | 80 | Transparent | - |
| | | 0.233 | 70 | Transparent | - |
| | | 0.466 | 70 | Transparent | - |
| | 7 % | 0.195 | 100 | Transparent | 1.05 |
| | | 0.279 | 70 | Transparent | - |
| | | 0.558 | 70 | Transparent | - |
| 30 L | 5 % | 0.172 | 24,000 | Not transparent | 1.05 ~ 1.06 |
| 40 L | | 0.196 | 28,000 | Transparent | 1.05 ~ 1.06 |
| 70 L | | 0.196 | 49,000 | Transparent | 1.05 ~ 1.06 |

In case of the ophthalmic composition containing 7% (w/v) or more of recoflavone with the ratio of molar concentration of sodium hydroxide by not less than 1.05, a clear dissolution of the active ingredient is not difficult to prepare because the molar concentration of sodium hydroxide is in the range of not less than 0.19M even though total volume of purified water is applied.

In case of the ophthalmic composition containing 3% (w/v) recoflavone, it is not also difficult to obtain a clear dissolution of the active ingredient which corresponds to the opposite case.

However, in case of the ophthalmic composition containing 5% (w/v) or 6% (w/v) recoflavone, the molar concentration of the sodium hydroxide aqueous solution may be out of the range of not more than 0.16 M or not less than 0.19 M depending on the applied amount of purified water first in the process. Unexpectedly, if recoflavone is not dissolved clearly due to out of the above range at this time, even though purified water is added again to adjust the molar concentration to not more than 0.16 M, it is too difficult to obtain a clear solution of recoflavone even after stirring for several hours. This would cause a big trouble in the manufacturing process of an eye drop.

In the present invention, in (S1) step, recoflavone, a hydrate, anhydride, or solvate thereof may be dissolved transparently in the ophthalmic composition in an amount of 5% (w/v) or more as recoflavone and specifically it may be dissolved transparently in the ophthalmic composition in an amount of 5% to 10% (w/v) as recoflavone.

In the present invention, the pH-adjusting agent used in (S2) step may be an acidifying agent. Specifically, when an alkalizing agent is used for dissolving recoflavone in (S1) step, the pH of the solution is increased to 10 or higher. Therefore, in (S2) step, an acidifying agent is added to adjust the pH to be in a range of 4 to 8 in consideration of the properties of an eye drop administered directly to eyes and the physicochemical properties of recoflavone.

In the present invention, in (S3) step, when one or more pharmaceutical additives are added, a permeation enhancer may be added to be 0.1 to 5.0% (w/v) in the ophthalmic composition, a tonicity agent to be 0.01 to 1.0% (w/v) in the ophthalmic composition, and a pH-adjusting agent to be 0.01 to 3.0% (w/v) in the ophthalmic composition. In this case, specifically the pH-adjusting agent comprises 0.01 to 2.5% (w/v) of an alkalizing agent and 0.001 to 0.5% (w/v) of an acidifying agent.

In the present invention, in (S3) step, when one or more pharmaceutical additives are added, a buffering agent may be added to be 0 to 3.0% (w/v) in the ophthalmic composition, and a preservative is to be 0 to 0.1% (w/v) in the ophthalmic composition.

In the present invention, the permeation enhancer may be one or more selected from polysorbate, polyoxyethylene alkyl ether, macrogol 15 hydroxystearate, polyoxyl glyceride, tocopheryl polyethylene glycol succinate, poloxamer and polyoxyethylene castor oil derivative, and specifically, one or more selected from polysorbate 80, polyoxyl 10 oleyl ether, solutol HS15, lauroyl polyoxyl glyceride, vitamin E-TPGS, poloxamer 407 and polyoxyl 35 castor oil.

In the present invention, the tonicity agent may be one or more selected from nonionic compounds consisting of mannitol, glycerin, propylene glycol, polyethylene glycol, maltose, sucrose, sorbitol, trehalose and glucose or ionic compounds consisting of sodium chloride, sodium nitrate and potassium nitrate.

In the present invention, a step of mixing by adding a viscosity agent to the solution obtained through the (S3) step may be further included before the (S4). This is not an essential step, and 0 to 3.0% (w/v) of a viscosity agent may be added and mixed.

In the present invention, before the (S4) step, the process of adding purified water to the solution obtained through (S3) step and checking the pH in the range of 4 to 8 may be further included.

Specifically, after checking the pH in the range of 4 to 8, a pH-adjusting agent may be added to adjust, if necessary.

The details mentioned about the ophthalmic composition for the treatment of dry eye disease, the ophthalmic composition in which the content of an alkalizing agent is adjusted, and the method for preparing the ophthalmic composition for the treatment of dry eye disease according to the present invention are equally applied as long as they do not contradict each other. The description about the ophthalmic composition for the treatment of dry eye disease and the ophthalmic composition in which the content of an alkalizing agent is adjusted can all be applied to the method for preparing the ophthalmic composition for the treatment of dry eye disease.

## Ophthalmic composition which provides the pH stability

In addition, the present invention provides an ophthalmic composition which provides the pH stability even without a buffering agent, if necessary.

Specifically, the present invention provides an ophthalmic composition for the treatment of dry eye disease, the composition containing 5% (w/v) or more of recoflavone or a pharmaceutically acceptable salt thereof, a hydrate, anhydride or solvate thereof,

comprising a pH-adjusting agent in an amount of 0.01 to 3.0 % (w/v); and maintaining the pH in a range of 4 to 8 under long-term storage conditions (25°C/60%RH). In this case, more specifically, the pH-adjusting agent may comprise 0.01 to 2.5% (w/v) of an alkalizing agent and 0.001 to 0.5% (w/v) of an acidifying agent.

In the present invention, the alkalizing agent is sodium hydroxide or potassium hydroxide.

In the present invention, the acidifying agent is one or more selected from the group of hydrochloric acid, lactic acid, acetic acid, phosphoric acid, boric acid and citric acid.

In the present invention, the ophthalmic composition which provides the pH stability maintains the pH in a range of 4 to 8 under long-term storage conditions (25°C/60%RH), specifically, the pH in a range of 5 to 7.

Specifically, the pH stability was confirmed since the pH was maintained in the range of 4 to 8, specifically 5

to 7, for 18 months under long-term storage conditions

(25°C/60%RH)

The details mentioned about the ophthalmic composition for the treatment of dry eye disease and the ophthalmic composition which provides the pH stability are equally applied as long as they do not contradict each other. The description about the ophthalmic composition for the treatment of dry eye disease can all be applied to the ophthalmic composition which provides the pH stability.

## Usage of ophthalmic composition

In addition, the present invention provides a method for the administration of an ophthalmic composition.

Specifically, the present invention provides an ophthalmic composition for the treatment of dry eye disease, the composition containing 5% (w/v) or more of recoflavone or a pharmaceutically acceptable salt thereof, a hydrate, anhydride or solvate thereof; and

comprising a permeation enhancer in an amount of 0.1 to 5.0 % (w/v),
for the administration of 1 to 2 drops at a time, 3 times or more a day.

For the ophthalmic composition of the present invention, it is advantageous that the active ingredient is sufficiently exposed to the eyes so that the drug can be delivered to the inside or outside of the keratoconjunctival cells of the eye in a certain amount or more for significant improvement of the overall symptoms of dry eye. Therefore, the method for the administration of the ophthalmic composition of the present invention is preferable to instill 1 to 2 drops at a time, 3 times or more a day.

Specifically, 1 drop at a time may be instilled 3 times or more, 4 times or more, 5 times or more, or 6 times or more, and more specifically, 1 drop at a time may be instilled 3 to 6 times.

In the present invention, the permeation enhancer may be one or more selected from polysorbate, polyoxyethylene alkyl ether, macrogol 15 hydroxystearate, polyoxyl glyceride, tocopheryl polyethylene glycol succinate, poloxamer and polyoxyethylene castor oil derivative, and specifically, one or more selected from polysorbate 80, polyoxyl 10 oleyl ether, solutol HS15, lauroyl polyoxyl glyceride, vitamin E-TPGS, poloxamer 407 and polyoxyl 35 castor oil.

In the present invention, the concentration of recoflavone is preferably not less than 5% (w/v) and not more than 10% (w/v). More than 10% (w/v) of recoflavone causes many difficulties involved in physical properties such as the osmolarity of an eye drop and practical problems such as the cost of products.

The ophthalmic composition according to the present invention may be administered in combination with other eye drops, and in this case, may be administered at an interval of at least 5 minutes.

The details mentioned about the ophthalmic composition for the treatment of dry eye disease and the usage of the ophthalmic composition are equally applied as long as they do not contradict each other. The description about the ophthalmic composition for the treatment of dry eye disease can all be applied to the usage of the ophthalmic composition.

Hereinafter, preferred Examples are presented to help the understanding of the present invention. However, the following Examples are provided for easier understanding of the present invention, and the content of the present invention is not limited by the Examples.

## Examples 1 to 5 and Comparative Examples 1 to 3: Preparation of ophthalmic compositions

An ophthalmic composition was prepared according to the ratio shown in Table 3 below. While stirring an appropriate amount of purified water, sodium hydroxide was added to the purified water to dissolve, and then recoflavone monohydrate was added to dissolve transparently. After the dissolution, hydrochloric acid was added to the dissolved solution to adjust to pH 4 to 8, and then a permeation enhancer, a tonicity agent, a buffering agent and a preservative were added to dissolve. A viscosity agent was separately put into in an appropriate amount of purified water and dissolved by heating or by keeping at room temperature depending on the type, and then added to the solution described above and mixed. However, the input order of a preservative, a permeation enhancer, a viscosity agent and a tonicity agent may be changed depending on the type and amount thereof. A viscosity agent, a buffering agent and a preservative may be omitted, if necessary. After checking if the pH was 4 to 8, the pH was adjusted using a pH-adjusting agent, if necessary. Additional purified water was applied to make sure the correct volume. The solution was passed through a filter of 0.22 μm or less to prepare a transparent ophthalmic composition.

[Table 3]

| Unit of concentration (%, w/v) | Comparative Exampl e 1 | Comparative Exampl e2 | Comparative Exampl e 3 | Exampl e 1 | Exampl e 2 | Exampl e 3 | Exampl e 4 | Exampl e 5 |
|---|---|---|---|---|---|---|---|---|
| Recoflavone | 5% | 3% | - | 5% | 5% | 5% | 5% | 5% |
| Polyoxyl 35 castor oil | - | 1% | 1% | 1% | - | 5% | 1% | 1% |
| Poloxamer 407 | - | - | - | - | 0.1% | - | 0.2% | - |
| Polyvinyl alcohol | - | - | - | - | 0.25% | - | - | - |
| Hydroxyethyl cellulose | - | - | - | - | 0.1% | - | - | - |
| Benzalkonium chloride | - | - | - | - | - | 0.01% | - | 0.01% |
| Sodium hydroxide | 0.55% | 0.33% | 0.55% | 0.55% | 0.55% | 0.55% | 0.55% | 0.55% |
| Hydrochloric acid | 0.03% | 0.02% | 0.50% | 0.03% | 0.03% | 0.03% | 0.03% | 0.03% |
| Sodium chloride | q.s. | q.s. | q.s. | q.s. | q.s. | q.s. | q.s. | q.s. |
| Glycerin | - | - | - | - | q.s. | - | - | - |
| Sorbitol | - | - | - | - | - | q.s. | - | - |
| Trehalose | | | | | - | q.s. | - | |
| Purified water | q.s. | q.s. | q.s. | q.s. | q.s. | q.s. | q.s. | q.s. |

**Experimental Example 1: Corneal permeability test**

In order to evaluate the degree of permeation of the ophthalmic composition in the keratoconjunctiva, the permeation rate was measured by using the cornea of minipig which is relatively uneasy to permeate and which allows for a corneal permeation test. In general, it is reported that the conjunctiva is 2-3 times easier to permeate compared to the cornea.

The *in vitro* test of corneal permeation for the ophthalmic compositions according to Comparative Examples and Examples was conducted by using a vertical Franz diffusion cell device. The cornea with the surrounding sclera for test was harvested from the eyes of minipigs that weighed 40 to 50 kg. In order to fix the cornea in Franz diffusion cell, the corneal region was only exposed at the center of receptor chamber. Then, the donor chamber was covered and fixed with a joint. Cautions were taken to remove the air from the area where the receptor chamber meets the cornea. 5mL of phosphate solution (PBS buffer) at pH 7.4 and a magnetic stirrer were put into the receptor chamber. 1mL of test solution was put into the donor chamber, which was then closed with a lid and sealed with a parafilm. During the permeability test, the stirrer kept at about 500 rpm, and the Franz diffusion cells kept at 36.5°C. At 0.5, 1, 2, 4 and 6 hours, 200 μL of each sample was taken and the same amount of phosphate solution (PBS buffer) was supplemented. The drug concentration in the collected samples was measured by using an HPLC analysis instrument. The concentration measured in each sample was converted to the permeation amount per time, and the amount of the diluent was corrected to calculate the cumulative permeation amount of recoflavone for 6 hours. The results of *in vitro* permeability test by using the cornea of minipig confirmed that the permeation enhancer which was contained in Examples increased the cumulative permeation amount of recoflavone.

The cumulative permeation amount per unit area in each individual sample was plotted with the average over sampling time, and the linear regression analysis was performed. The slope of equation was calculated as the average permeation rate (flux) of the drug for 6 hours. The relative permeation enhancement ratio was calculated as the ratio of the average permeation rate for 6 hours of each Example compared to Comparative Example 1 (see equation 1).

[Equation 1]

$$\text{Relative permeation enhancement ratio} = \frac{\text{Average permeation rate of each Example}}{\text{Average permeation rate of Comparative Example 1}}$$

Through the above method, the average cumulative permeation amount and the average permeation rate over time per unit area of the cornea, and the relative permeation enhancement ratio of the ophthalmic compositions of Examples 1 to 3 and Comparative Examples 1 and 2 were calculated and are shown in Table 4.

Referring to Table 4, Examples 1 to 3 confirmed that permeation of the drug was different depending on the presence or absence of a permeation enhancer at the same drug concentration compared to Comparative Example 1 which contains only the active ingredient like a conventional eye drop composition. In addition, Example 1 confirmed that the permeation was enhanced depending on the concentration of recoflavone at the same composition compared to Comparative Example 2.

In addition, it was confirmed that the permeability of Comparative Example 2 which had a lower concentration of recoflavone but had a permeation enhancer was higher than that of Comparative Examples 1.

The results showed that Example 1 had an average permeation rate of 27 $\mu g/cm^2/hr$ for 6 hours and so the relative permeation enhancement ratio increased 3.4 times compared to Comparative Example 1 without a permeation enhancer.

The improvement of the symptoms of dry eye *in vivo* was also comparatively evaluated in Experimental Examples described below, and the results showed that the overall improvement by Example 1 was significantly better than those by Comparative Example 1 without a permeation enhancer.

Therefore, in pursuit of the overall improvement of the symptoms of dry eye *in vivo* rather than simply getting better the laboratory data, it was confirmed that the relative permeation enhancement ratio for 6 hours in the cornea of minipig, compared to Comparative Example 1 without a permeation enhancer, should preferably be 3.0 or higher, or the cumulative permeation amount should preferably be 130 $\mu g/cm^2$ or more.

[Table 4]

| | Comparativ e Example 1 (n=6) | Comparativ e Example 2 (n=6) | Example 1 (n=6) | Example 2 (n=3) | Example 3 (n=3) |
|---|---|---|---|---|---|
| Cumulative permeation amount ($\mu g/cm^2$) | 43.21 | 81.14 | 149.53 | 315.09 | 252.31 |
| Ratio of cumulative permeation amount compared to Comparative Example 1 | 1.00 | 1.88 | 3.46 | 7.29 | 5.84 |
| Average permeation rate ($\mu g/cm^2/hr$) | 7.86 | 14.64 | 27.04 | 56.35 | 45.36 |
| Relative permeation enhancement ratio compared to Comparative Example 1 | 1.00 | 1.86 | 3.44 | 7.17 | 5.77 |

## Experimental Example 2: Tear secretion

It was evaluated the effect of the improved corneal permeation of the ophthalmic composition on the tear secretion in the experimental dry eye mouse model.

NOD.B10.H2$^b$ mice 12 to 14 weeks old (Jackson Laboratory, Bar Harbor, USA) were adapted for 7 days and were injected subcutaneously with 0.5 mg/0.2 mL scopolamine hydrobromide into hindquarters 4 times daily (8 am, 11 am, 2 pm, 5 pm) for 10 days with desiccation stress at below average 40% of ambient humidity. After checking the occurrence of dry eyes, the placebo (the composition without the active ingredient in Example 1), Comparative Example

1 and Example 1 were each instilled into both eyes at a dose of 5 μL, four times a day for 21 days.

To measure the tear volume, phenol red-impregnated cotton threads were placed in the lateral canthus for 20 seconds. The lengths of the thread in which tear was absorbed were converted into microliters (μL).

FIG. 2 shows the results of the tear secretion in the experimental dry eye mouse model by the method described above. Example 1 with the increased corneal permeation improved the tear volume, while Comparative Example 1 containing 5% (w/v) recoflavone as same as Example 1 showed little difference of the tear volume from that of the placebo.

**Experimental Example 3: Corneal Fluorescein Staining (CFS)**

It was evaluated the effect of the improved corneal permeation of the ophthalmic composition on the corneal fluorescence staining (CFS) in the experimental dry eye mouse model.

On the same condition of Experimental Example 2, Comparative Example 3, Comparative Example 1 and Example 1 were instilled for 10 days and 1 μL of 1% fluorescein reagent was applied into the eyes to stain the cornea and the eyes was washed with saline. Images were taken with a digital slit lamp to score and evaluate the degree of epithelial damage (the degree of fluorescence staining). The scores from 1 to 3 points for each five areas of cornea were given and added up.

FIG. 3 shows the results of the corneal fluorescence staining (CFS) as a direct index for the improvement of dry eye disease by the method described above. Example 1 showed significant reduction of the corneal fluorescence staining (CFS) which meant the symptoms of dry eye were significantly improved while Comparative Example 1 exhibited no improvement compared to the placebo (Comparative Example 3).

**Experimental Example 4: Evaluation of corneal surface irregularity**

It was evaluated the effect of the improved corneal permeation of the ophthalmic composition on the corneal surface irregularity in the experimental dry eye mouse model.

On the same conditions of experimental Example 2, Comparative Example 3, Comparative Example 1 and Example 1 were instilled for 10 days. The white ring images of the cornea surface were reflected and acquired from the fiber optic ring illuminator of a stereoscopic microscope (SZX7, Olympus). The corneal surface irregularity score was given in the reflected ring: 0 point no distortion; 1 point, distortion in 1 quarter of the ring; 2 points, distortion in 2 quarters; 3 points, distortion in 3 quarters; 4 points, distortion in all 4 quarters; 5 points, severe distortion.

The corneal surface irregularity was evaluated to check the effect on the corneal curing by the method described above (FIG. 4). Example 1 showed the reduction of the corneal surface irregularity by about 30% which indicated that the symptoms of dry eye were significantly improved while Comparative Example 1 did not show any difference from Comparative Example 3 as placebo.

**Experimental Example 5: Detachment of corneal epithelial cell**

It was evaluated the effect of the improved corneal permeation of the ophthalmic composition on the detachment of corneal epithelial cell in the experimental dry eye mouse model.

On the same conditions of Experimental Example 2, Comparative Example 3, Comparative Example 1 and Example 1 were instilled for 21 days and the animals were sacrificed.

The surgically excised periocular region and lacrimal gland were fixed in 10% formalin for 3 days and embedded in paraffin. The 5 μm tissue was cut with a microtome and dried for 1 hour on a slide. The tissue was prepared by the Hematoxylin-Eosin (H&E) staining method and the detached epithelial cell was evaluated in the 1 $mm^2$ of corneal region by a virtual microscope (Nanozoomer 2.0RS, Hamamatsu, Japan). In order to compare exactly the degree of improvement of the symptoms of dry eye, the eye tissue at the time right before the drug administration after dry eye was fully induced (initial) and the eye tissue of the normal mouse that was exposed to neither the drug or nor the dry environment stress for 10 days (normal eye) were compared together.

FIG. 5 shows the results of evaluating the detached corneal epithelial cell. While the detached epithelial cell was very little in the normal eye, the detachment was severely increased through the exposure to the dry eye-inducing environment for 10 days (initial). Though Comparative Example 1 exhibited a clear improvement compared to Comparative Example 3 as placebo, Example 1 showed a better result of recovery to the state of the normal eye which indicated that the detached epithelial cell was effectively reduced.

**Experimental Example 6: Conjunctival goblet cell density**

It was evaluated the effect of the improved corneal permeation of the ophthalmic composition on the density of goblet cells for the mucus secretion in the experimental dry eye mouse model.

On the same conditions of Experimental Example 5, the paraffin-embedded tissue was sectioned and put on a slide. The staining was performed according to the instructions of the PAS (Periodic-Acid-Schiff) kit and the density of goblet cells was evaluated in the 1 $mm^2$ of conjunctival region by a virtual microscope (Nanozoomer 2.0RS, Hamamatsu, Japan).

FIG. 6 shows the results of evaluating the density of conjunctival goblet cells by the method described above. The conjunctival goblet cells of the initial exhibited a significant reduction through the exposure to the dry eye-inducing environment for 10 days compared to the normal eye. Comparative Example 3 as placebo showed no recovery of the conjunctival goblet cells. Comparative Example 1 exhibited a clear improvement effect compared to the placebo, whereas Example 1 showed further improvement from Comparative Example 1 which exhibited a better result of recovery that was close to the level of the normal eye.

**Experimental Example 7: Distribution of mucins**

It was evaluated the effect of the improved corneal permeation of the ophthalmic composition on the distribution of mucins, a major component of mucus, in the experimental dry eye mouse model.

On the same conditions of Experimental Example 5, the paraffin-embedded tissue was sectioned and put on a slide. The staining was performed according to the instructions of the Alcian Blue Stain kit and it was evaluated in the 1 $mm^2$ of corneal and conjunctival regions by a virtual microscope (Nanozoomer 2.0RS, Hamamatsu, Japan).

FIGS. 7 and 8 show the distribution of mucins by the method described above. The mucins of the initial exhibited a significant reduction through the exposure to the dry eye-inducing environment for 10 days compared to the normal eye. The placebo showed no recovery of the mucins which are key components of mucus. Comparative Example 1 exhibited a clear improvement effect compared to Comparative Example 3 as placebo, whereas Example 1 showed further improvement from Comparative Example 1 which exhibited a better result of recovery that was close to the level of the normal eye. These were consistent with the results of the density of goblet cells for the mucin secretion in Experimental Example 6.

**Experimental Example 8: Clinical study of the ophthalmic composition**

It was evaluated the clinical efficacy and safety of the ophthalmic composition containing 5% (w/v) of recoflavone (Example 5) in patients with dry eye disease within the inclusion criteria. It was a multi-center, randomized, double-blind, placebo-controlled, phase 2 clinical study. Specifically, a run-in period was employed after screening patients who are within the inclusion criteria of this clinical study. The patients who kept an acceptable level of medical compliance were randomized into three groups (placebo group, test 3 times-a-day group, and test 6 times-a-day group). Thirty-three subjects were assigned to each group. The efficacy and safety were evaluated by administering 1 drop, 3 times or 6 times a day, for up to 12 weeks. The change of the total corneal staining score (TCSS) from the baseline was measured by fluorescein for the efficacy evaluation. In addition, adverse events, vital signs, laboratory tests, electrocardiography, and ophthalmic tests were performed to evaluate the safety at each visit.

Table 5 and FIG. 10 show the amount of change in the total corneal staining score (TCSS) as the primary efficacy variable. As the administration time continued, the difference from the placebo group tended to increase. When a t-test was performed at a level of $p<0.05$ to verify the significance of difference between the groups, the results showed that the difference was not significant until Week 2 after administration, but at Week 8, both the test 3 times-a-day group and the test 6 times-a-day group showed a significant difference from the placebo group. However, the difference was not significant between the test 3 times-a-day group and the test 6 times-a-day group in both Week 2 and Week 8.

Based on the results of the clinical study above, the novel recoflavone-containing ophthalmic composition containing a permeation enhancer in the present invention was evaluated to show a practical and significant improvement of dry eye disease in human, which could not be overcome by the conventional inventions.

[Table 5]

| | Placebo group | 3-time administration group | 6-time administration group |
|---|---|---|---|
| Week 2 | -1.36±1.80 | -1.85±1.72 | -1.91±2.69 |
| Week 8 | -2.22±2.17 | -3.45±1.95* | -3.35±2.24* |
| *Significantly different from the placebo group at a level of p<0.05. | | | |

**Experimental Example 9: Evaluation of pH stability of the ophthalmic composition**

The pH of the ophthalmic solutions of Example 5 and the placebo were evaluated under long-term storage conditions (25°C/60% RH) and the results are shown in FIG. 11.

In spite of no buffering agent in Example 5, the pH was stable within the range of 6 to 7, which was the initial pH, for 18 months under long-term storage conditions, whereas the pH of the placebo containing no recoflavone showed a continuous decline.

A buffering agent has been used in most eye drops to maintain stable physicochemical properties, especially pH, during the shelf life of products. Recently, it was suggested that a buffering agent could cause some problems. Surprisingly, it was confirmed that the ophthalmic compositions of the present invention can keep the pH stable under long-term storage conditions (25°C/60% RH) even though the composition does not contain a buffering agent, if necessary.

As shown above, a specific part of the present invention was described in details, and for those of ordinary skill in the related art, this specific technology is only a preferred embodiment, and it is clear that the scope of the present invention is not limited thereto. Therefore, the substantial scope of the invention will be defined by the appended claims and their equivalents.

## Claims

1. An ophthalmic composition for the treatment of dry eye disease, the composition comprising 5% (w/v) or more of recoflavone or a pharmaceutically acceptable salt thereof, a hydrate, anhydride or solvate thereof;

   a permeation enhancer in an amount of 0.1 to 5.0 % (w/v); and
   a tonicity agent in an amount of 0.01 to 1.0% (w/v).

2. The ophthalmic composition for the treatment of dry eye disease of claim 1, wherein the ophthalmic composition for the treatment of dry eye disease further comprises at least one of a viscosity agent, a buffering agent, a pH-adjusting agent and a preservative.

3. The ophthalmic composition for the treatment of dry eye disease of claim 1, wherein the cumulative permeation amount of recoflavone measured in a Franz diffusion cell in the cornea of minipig for 6 hours is 130 $\mu g/cm^2$ or more or 3 times or more compared to an ophthalmic composition that does not contain a permeation enhancer.

4. The ophthalmic composition for the treatment of dry eye disease of claim 1, wherein the permeation enhancer contains at least one selected from polysorbate, polyoxyethylene alkyl ether, macrogol 115 hydroxystearate, polyoxyl glyceride, tocopheryl polyethylene glycol succinate, poloxamer and polyoxyethylene castor oil derivatives.

5. The ophthalmic composition for the treatment of dry eye disease of claim 4, wherein the permeation enhancer comprises at least one selected from polysorbate 80, polyoxyl 10 oleyl ether, solutol HS15, lauroyl polyoxyl glyceride, vitamin E-TPGS, poloxamer 407 and polyoxyl 35 castor oil.

6. The ophthalmic composition for the treatment of dry eye disease of claim 1, wherein the tonicity agent comprises at least one selected from nonionic compounds consisting of mannitol, glycerin, propylene glycol, polyethylene glycol, maltose, sucrose, sorbitol, trehalose and glucose or ionic compounds consisting of sodium chloride, sodium nitrate and potassium nitrate.

7. An ophthalmic composition for the treatment of dry eye disease, the composition comprising 5% (w/v) or more of recoflavone or a pharmaceutically acceptable salt thereof, a hydrate, anhydride or solvate thereof; and

   a permeation enhancer in an amount of 0.1 to 5.0 % (w/v);
   1 to 2 drops are administered at a time and at least 3 times a day.

8. The ophthalmic composition for the treatment of dry eye disease of claim 7, wherein the ophthalmic composition is administered 1 drop at a time, 3 times to 6 times a day.

【FIG. 1】

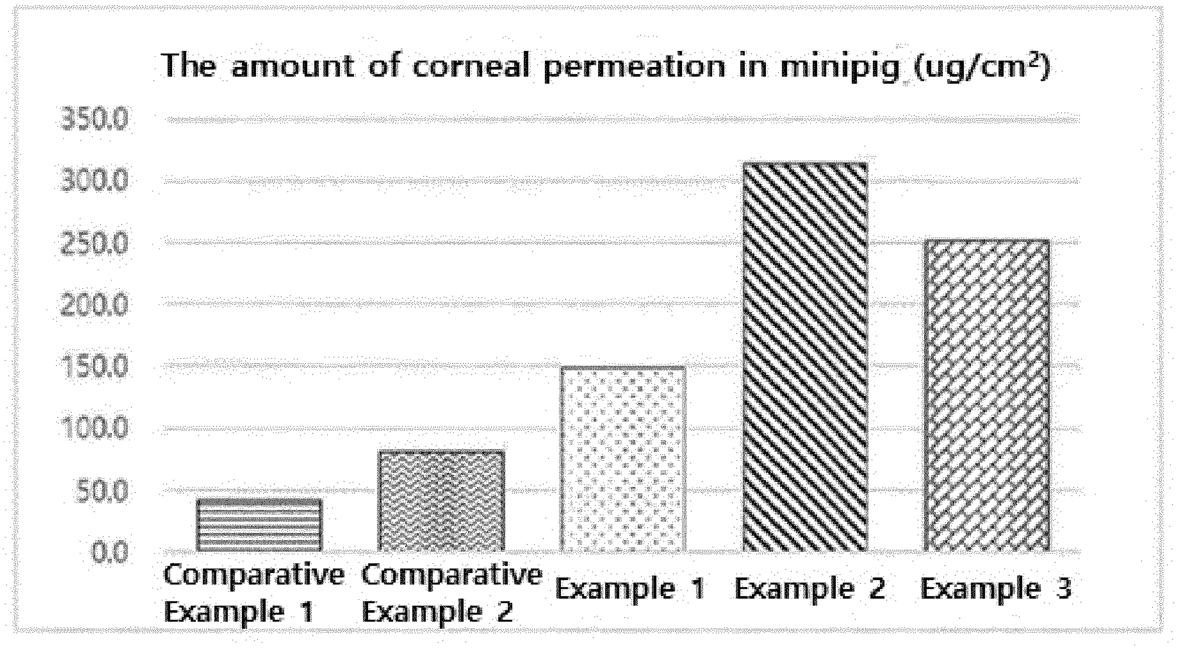
The amount of corneal permeation in minipig (ug/cm²)
350.0
300.0
250.0
200.0
150.0
100.0
50.0
0.0
Comparative Example 1
Comparative Example 2
Example 1
Example 2
Example 3

【FIG. 2】

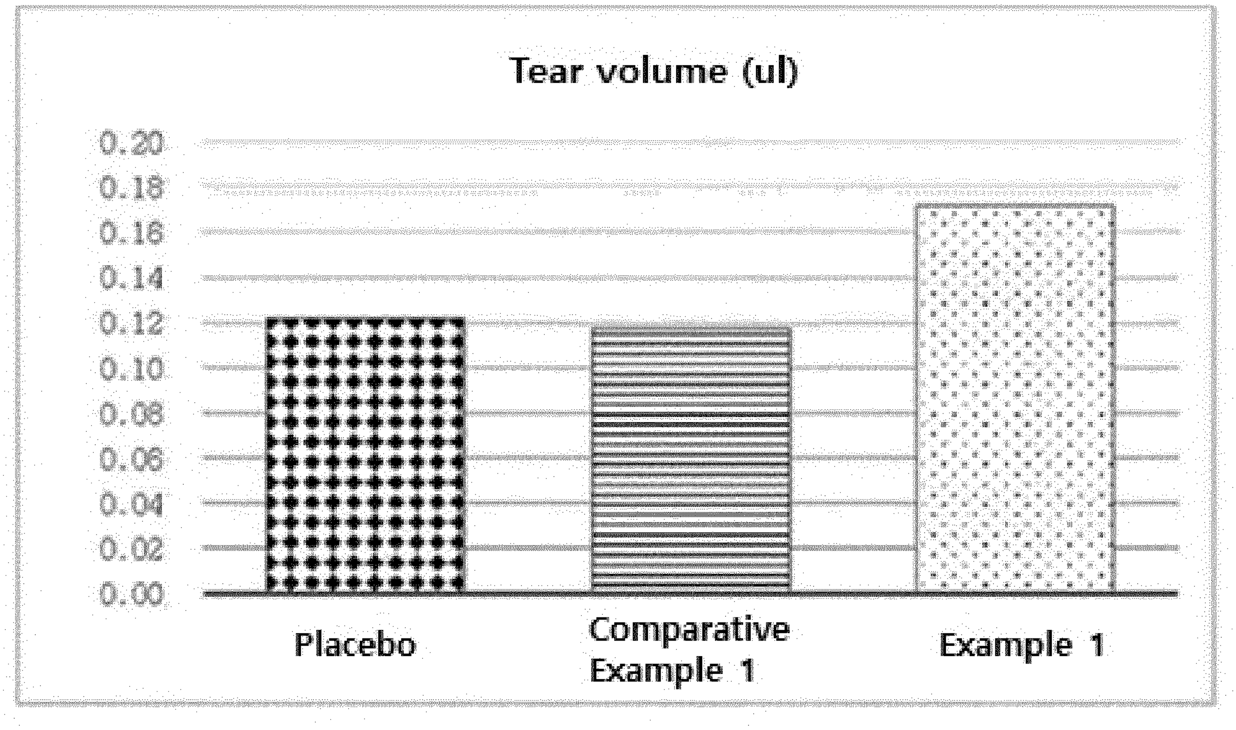
Tear volume (ul)
0.20
0.18
0.16
0.14
0.12
0.10
0.08
0.06
0.04
0.02
0.00
Placebo
Comparative Example 1
Example 1

【FIG. 3】

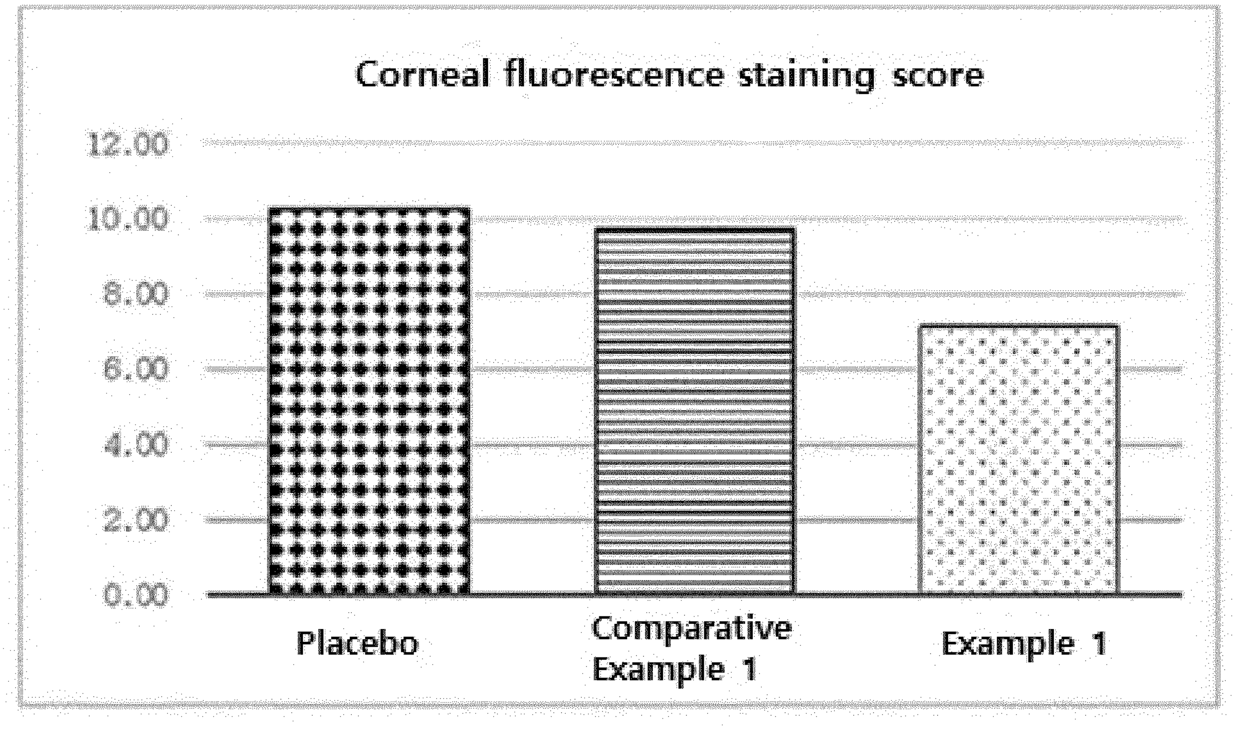
Corneal fluorescence staining score
12.00
10.00
8.00
6.00
4.00
2.00
0.00
Placebo
Comparative Example 1
Example 1

【FIG. 4】

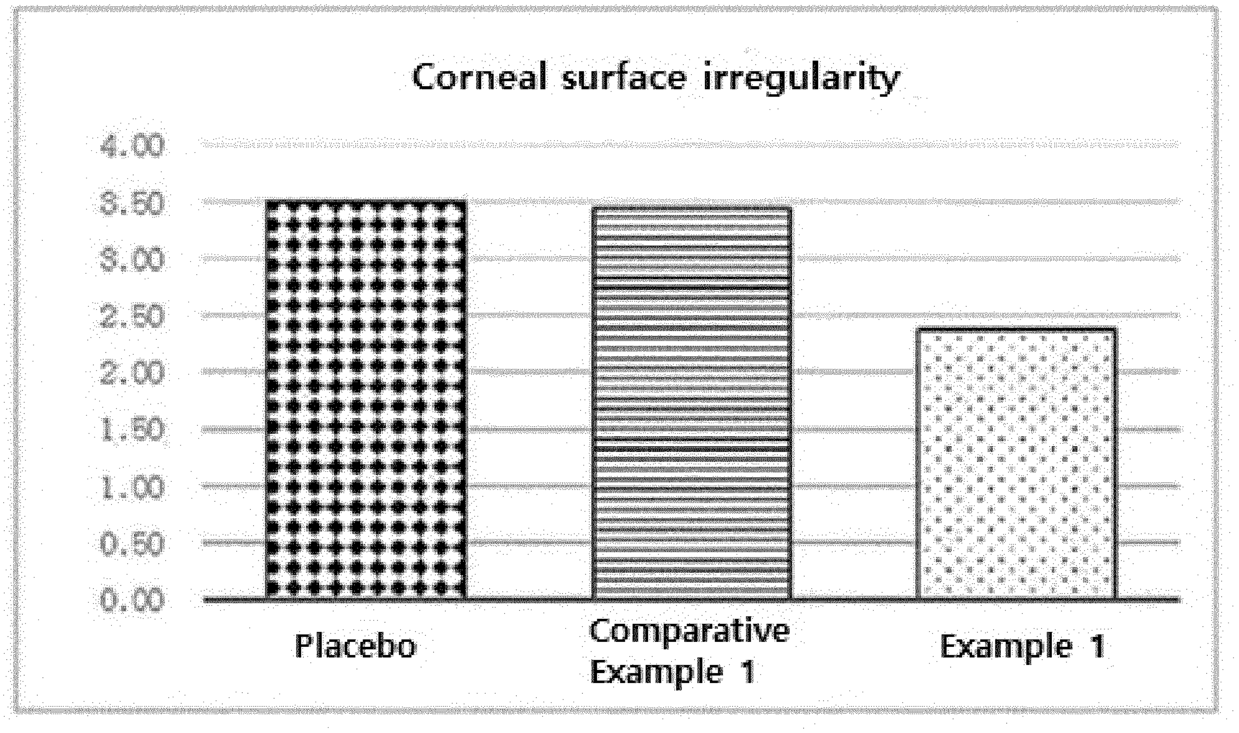
Corneal surface irregularity
4.00
3.50
3.00
2.50
2.00
1.50
1.00
0.50
0.00
Placebo
Comparative Example 1
Example 1

【FIG. 5】

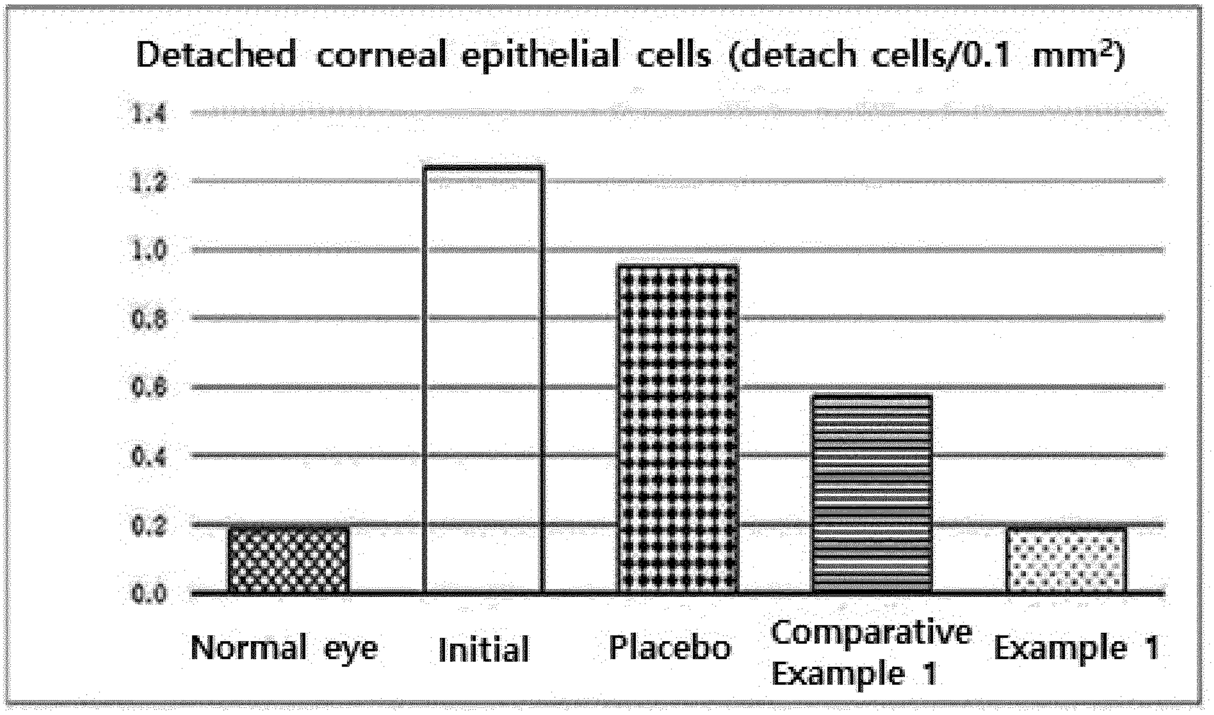
Detached corneal epithelial cells (detach cells/0.1 mm²)
1.4
1.2
1.0
0.8
0.6
0.4
0.2
0.0
Normal eye
Initial
Placebo
Comparative Example 1
Example 1

【FIG. 6】

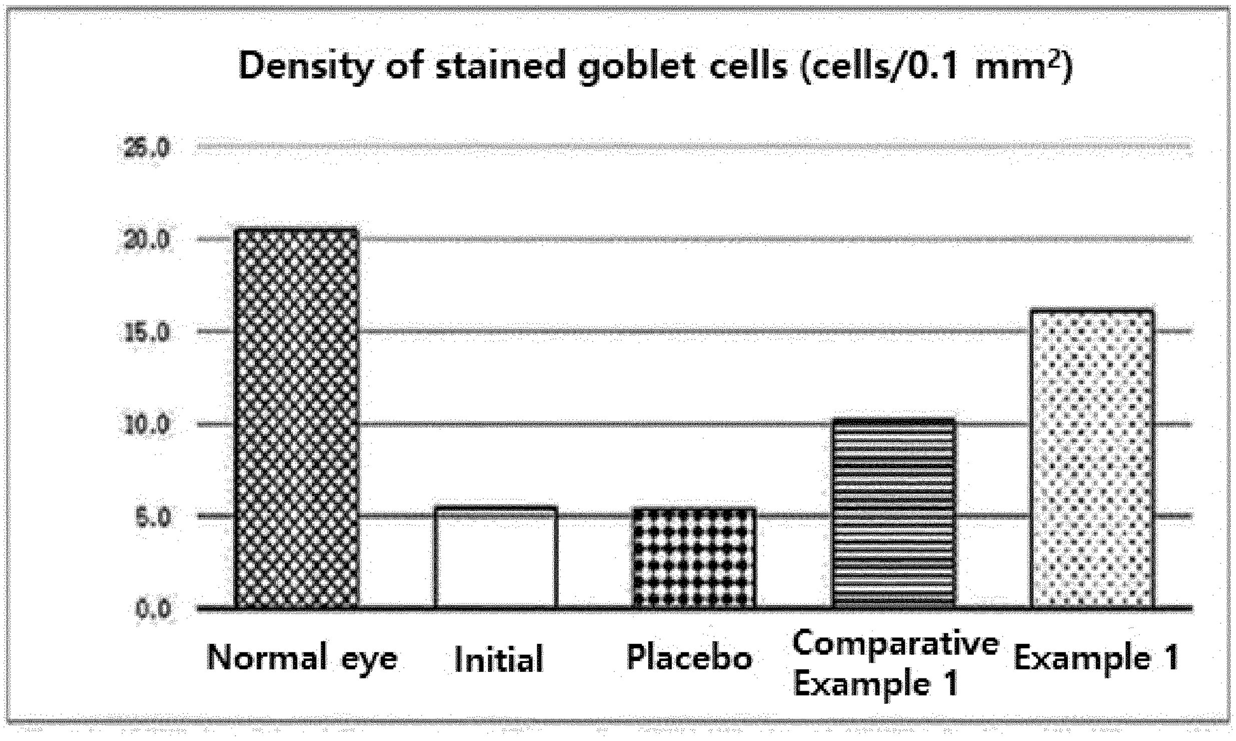
Density of stained goblet cells (cells/0.1 mm²)
25.0
20.0
15.0
10.0
5.0
0.0
Normal eye
Initial
Placebo
Comparative Example 1
Example 1

【FIG. 7】

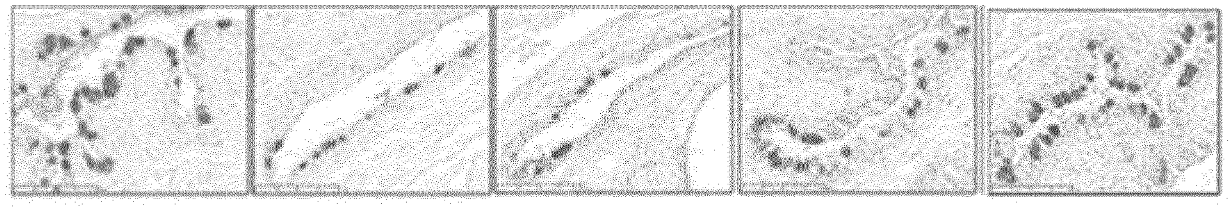

(a) Normal eye (b) Initial (c) Placebo (d) Comparative Example 1 (e) Example 1

【FIG. 8】

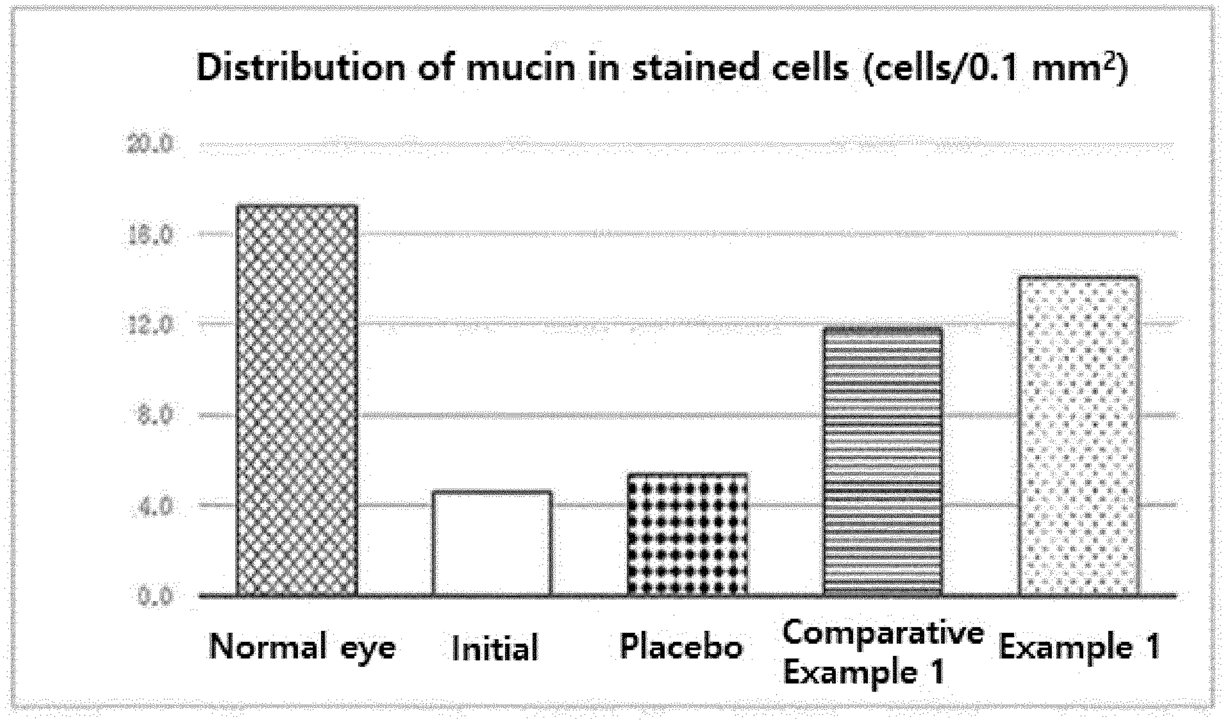
Distribution of mucin in stained cells (cells/0.1 mm2)
20.0
16.0
12.0
8.0
4.0
0.0
Normal eye
Initial
Placebo
Comparative Example 1
Example 1

【FIG. 9】

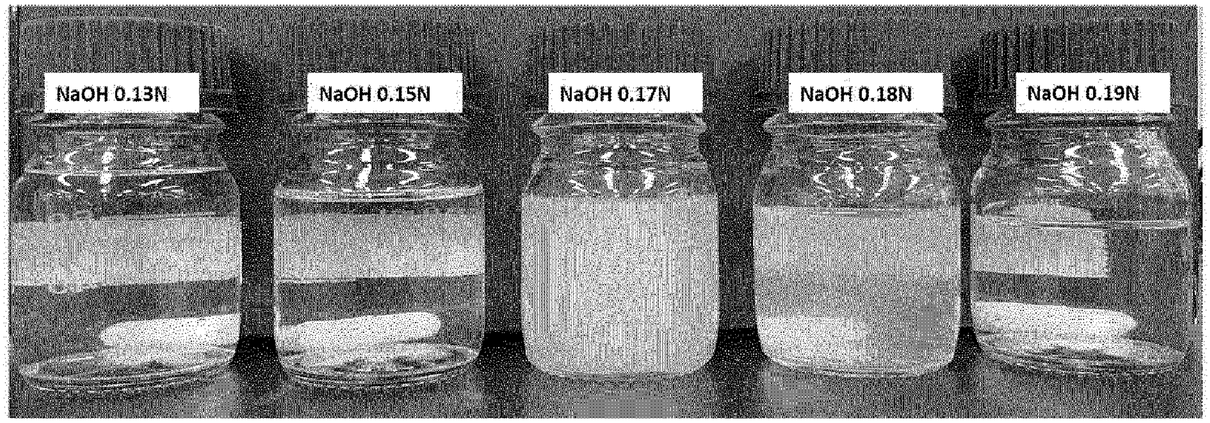

【FIG. 10】

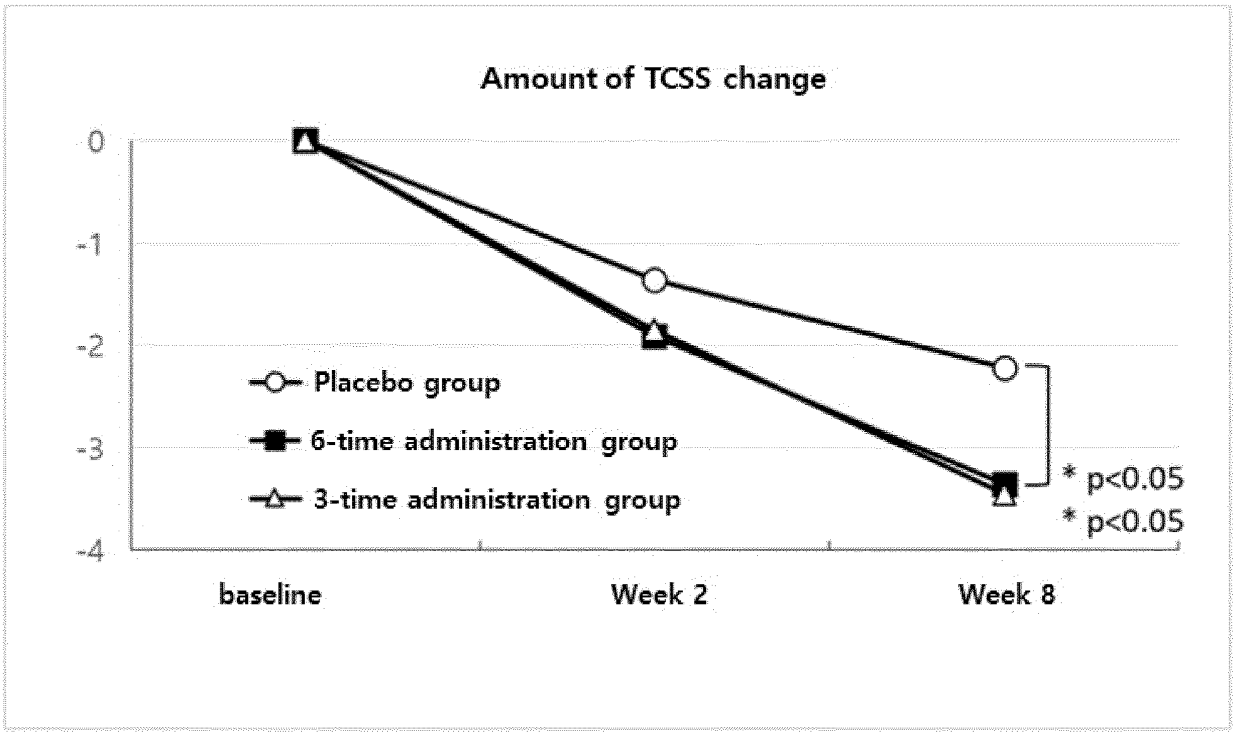
Amount of TCSS change
0
-1
-2
-3
-4
Placebo group
6-time administration group
3-time administration group
* p<0.05
* p<0.05
baseline
Week 2
Week 8

【FIG. 11】

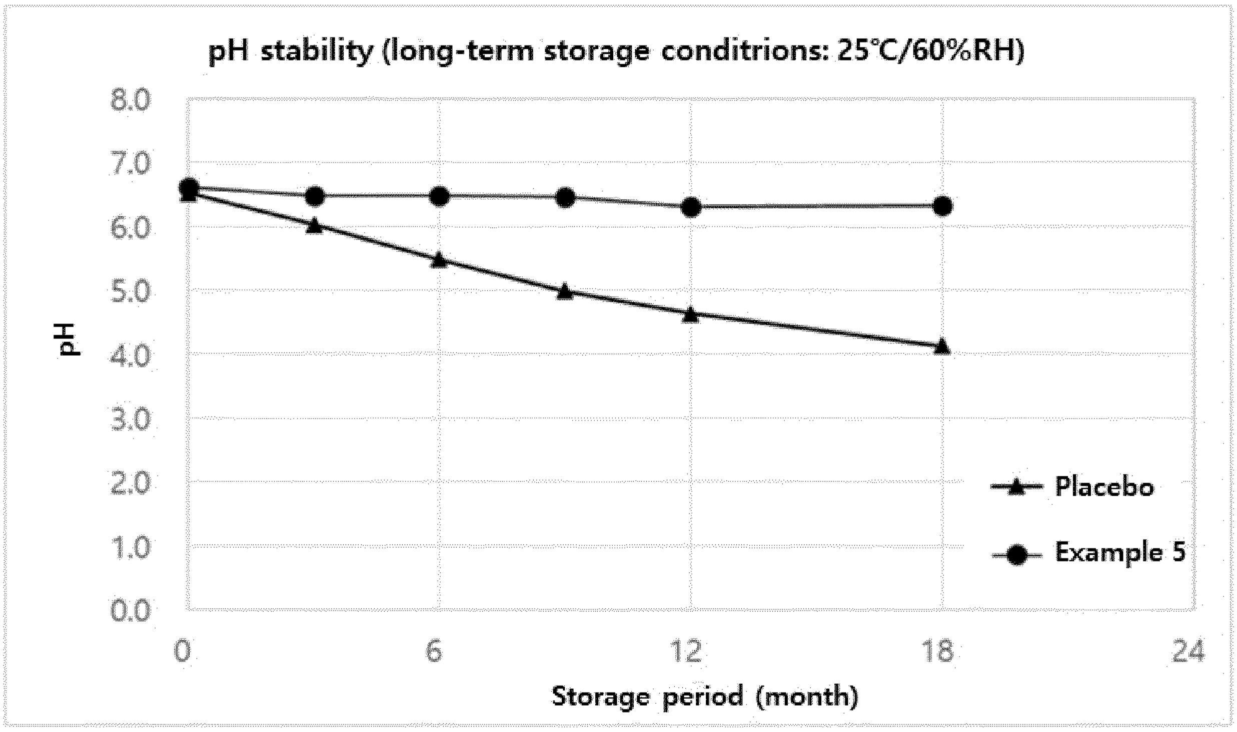
pH stability (long-term storage conditrions: 25°C/60%RH)
pH
8.0
7.0
6.0
5.0
4.0
3.0
2.0
1.0
0.0
0
6
12
18
24
Storage period (month)
Placebo
Example 5

## INTERNATIONAL SEARCH REPORT

International application No.
**PCT/KR2022/011567**

**A. CLASSIFICATION OF SUBJECT MATTER**

**A61K 9/00**(2006.01)i; **A61K 9/08**(2006.01)i; **A61K 47/26**(2006.01)i; **A61K 47/10**(2006.01)i; **A61K 47/44**(2006.01)i; **A61K 31/352**(2006.01)i; **A61P 27/02**(2006.01)i; **A61P 27/04**(2006.01)i

According to International Patent Classification (IPC) or to both national classification and IPC

**B. FIELDS SEARCHED**

Minimum documentation searched (classification system followed by classification symbols)

A61K 9/00(2006.01); A61K 31/353(2006.01); A61K 31/7048(2006.01); A61K 33/22(2006.01); A61K 47/10(2006.01); A61K 47/18(2006.01); A61K 47/26(2006.01); A61K 47/44(2006.01); C07D 311/30(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 레코플라본(recoflavone), 안구건조증(xerophthalmia), 점안제(eye drop), 침투촉진제(permeation enhancer), 등장화제(isotonic agent)

**C. DOCUMENTS CONSIDERED TO BE RELEVANT**

| Category* | Citation of document, with indication, where appropriate, of the relevant passages | Relevant to claim No. |
|---|---|---|
| DY | KR 10-2008-0107542 A (DONG-A PHARM. CO., LTD.) 11 December 2008 (2008-12-11)<br>See paragraph [0065]; the formulation examples; and claim 8. | 1-8 |
| Y | KR 10-2021-0038600 A (JOHNSON & JOHNSON SURGICAL VISION, INC.) 07 April 2021 (2021-04-07)<br>See paragraphs [0025], [0218], [0219] and [0231]; table 1; and claims 1 and 7-11. | 1-8 |
| A | KR 10-2020-0099547 A (SANTEN PHARMACEUTICAL CO., LTD.) 24 August 2020 (2020-08-24)<br>See entire document. | 1-8 |
| A | US 2010-0209539 A1 (RENNEBERG, J.) 19 August 2010 (2010-08-19)<br>See entire document. | 1-8 |
| PX | KR 10-2356603 B1 (GL PHARMTECH CORP.) 08 February 2022 (2022-02-08)<br>See entire document. | 1-8 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 November 2022** | **14 November 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2022/011567**

| Patent document cited in search report | | | Publication date (day/month/year) | Patent family member(s) | | | Publication date (day/month/year) |
|---|---|---|---|---|---|---|---|
| KR | 10-2008-0107542 | A | 11 December 2008 | AT | 529417 | T | 15 November 2011 |
| | | | | AU | 2008-260829 | A1 | 11 December 2008 |
| | | | | AU | 2008-260829 | B2 | 08 November 2012 |
| | | | | BR | PI0812494 | A2 | 16 June 2015 |
| | | | | CA | 2689136 | A1 | 11 December 2008 |
| | | | | CA | 2689136 | C | 13 November 2012 |
| | | | | CN | 101679341 | A | 24 March 2010 |
| | | | | CN | 101679341 | B | 06 June 2012 |
| | | | | DK | 2170857 | T3 | 05 December 2011 |
| | | | | EP | 2170857 | A2 | 07 April 2010 |
| | | | | EP | 2170857 | B1 | 19 October 2011 |
| | | | | ES | 2375184 | T3 | 27 February 2012 |
| | | | | JP | 2010-529112 | A | 26 August 2010 |
| | | | | JP | 5295230 | B2 | 18 September 2013 |
| | | | | KR | 10-0930467 | B1 | 08 December 2009 |
| | | | | MX | 2009013062 | A | 18 January 2010 |
| | | | | PT | 2170857 | E | 12 December 2011 |
| | | | | US | 2010-0130448 | A1 | 27 May 2010 |
| | | | | US | 8203011 | B2 | 19 June 2012 |
| | | | | WO | 2008-150085 | A2 | 11 December 2008 |
| | | | | WO | 2008-150085 | A3 | 26 February 2009 |
| KR | 10-2021-0038600 | A | 07 April 2021 | AU | 2019-311849 | A1 | 21 January 2021 |
| | | | | BR | 112021000634 | A2 | 06 April 2021 |
| | | | | CA | 3106898 | A1 | 30 January 2020 |
| | | | | CN | 112654359 | A | 13 April 2021 |
| | | | | EP | 3829610 | A1 | 09 June 2021 |
| | | | | JP | 2021-532121 | A | 25 November 2021 |
| | | | | US | 2021-000897 | A1 | 07 January 2021 |
| | | | | WO | 2020-021481 | A1 | 30 January 2020 |
| KR | 10-2020-0099547 | A | 24 August 2020 | CN | 111712238 | A | 25 September 2020 |
| | | | | WO | 2019-117252 | A1 | 20 June 2019 |
| US | 2010-0209539 | A1 | 19 August 2010 | AU | 2007-323468 | A1 | 29 May 2008 |
| | | | | CA | 2670409 | A1 | 29 May 2008 |
| | | | | EP | 2094287 | A1 | 02 September 2009 |
| | | | | WO | 2008-061536 | A1 | 29 May 2008 |
| KR | 10-2356603 | B1 | 08 February 2022 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 100447918
- KR 100327621
- KR 100644928
- KR 100930467

### Non-patent literature cited in the description


- TFOS DEWS II introduction. *Ocul. Surf.,* 2017, vol. 15, 269-275
- *J. Korean Med. Assoc.,* 2018, vol. 61 (6), 352-364
- TFOS DEWS II introduction. *Ocul. Surf,* 2017, vol. 15, 269-275
- *Invest. Ophthalmol. Vis. Sci.,* 2014, vol. 55 (10), 6569-6574
- *Cornea,* 2017, vol. 36 (6), 712-718
- *Br. J. Ophthalmol.,* 2010, vol. 94, 1519-1522
- *Investigative Ophthalmology and Visual Science,* 2014, vol. 55 (10), 6565-6574